## Europäisches Patentamt

## European Patent Office

## Office européen des brevets

(19)

(11) Veröffentlichungsnummer: **0 238 567**

**B1**

(12) **EUROPÄISCHE PATENTSCHRIFT**

(45) Veröffentlichungstag der Patentschrift:
06.12.89

(21) Anmeldenummer: 86905722.4

(22) Anmeldetag: 23.09.86

(86) Internationale Anmeldenummer:
PCT/DE 86/00388

(87) Internationale Veröffentlichungsnummer:
WO 87/01861 (26.03.87 Gazette 87/07)

(51) Int. Cl.⁴: **G 21 K 5/02**, A 61 L 2/08,
A 23 L 3/26

(54) **VERFAHREN UND VORRICHTUNG ZUR BESTRAHLUNG VON BESTRAHLUNGSGUT MITTELS IONISIERENDER STRAHLUNG.**

(30) Priorität: 23.09.85 DE 3533825

(43) Veröffentlichungstag der Anmeldung:
30.09.87 Patentblatt 87/40

(45) Bekanntmachung des Hinweises auf die Patenterteilung:
06.12.89 Patentblatt 89/49

(84) Benannte Vertragsstaaten:
AT BE CH DE FR GB IT LI NL SE

(56) Entgegenhaltungen:
DE-C-1 159 350
FR-A-2 252 633
FR-A-2 298 166

Atomkernenergie Kerntechnik, Band 34, Nr. 4, 1979, München (DE), K.H. Tetzlaff:
"Strahlensterilisation: Fortschritte mit neuem Anlagentyp", Seiten 305-30

(73) Patentinhaber: TETZLAFF, Karl- Heinz,
Mörikestrasse 6, D-6233 Kelkheim (DE)

(72) Erfinder: TETZLAFF, Karl- Heinz, Mörikestrasse 6,
D-6233 Kelkheim (DE)

(74) Vertreter: Steinmann, Otto C., c/o Münich, Neidl-Stippler, Schiller Willibaldstr. 36, D-8000
München 21 (DE)

# Beschreibung

## Technisches Gebiet

Die Erfindung betrifft ein Verfahren und eine Vorrichtung zur Bestrahlung von Bestrahlungsgut, das zu großen Transporteinheiten verpackt ist, mittels ionisierender Strahlungsquellen und insbesondere mittels Röntgen- oder GammaStrahlungsquellen.

Derartige Verfahren werden beispielsweis zum Sterilisieren von medizinischen Einwegartikeln und zur Behandlung von Lebensmittteln verwendet.

## Stand der Technik

Eine industrielle Bestrahlungsanlage soll möglichst große Packungseinheiten, bzw. Transporteinheiten, möglichst gleichmäßig mit hohem Strahlungswirkungsgrad bestrahlen. Als Strahlungswirkungsgrad bezeichnet man das Verhältnis von ausgenutzter Strahlung zur gesamten emittierten Strahlung. Der ausgenutzte Anteil bezieht sich dabei auf die Minimaldosis innerhalb einer Transporteinheit, der darüberliegende emitierte Dosisanteil muß daher als verloren angesehen werden.

Aus der DE-PS-2 358 652 und aus ATOM-KERNERNERGIE/KERNTECHNIK 34 (1979) 4, 305/308 ist eine Vorrichtung bekannt, bei der säulenförmig übereinander angeordnete große Transporteinheiten kreisförmig um eine stabförmige Strahlungsquelle angeordnet sind, wobei sich die Säulen um ihre jeweilige eigene Symmetrieachse drehen. Seitlich des Strahlenganges der Strahlungsquelle und der Drehachse der Säulen sind Abschirmelemente angeordnet, die den peripheren Bereich des Bestrahlungsgutes teilweise abschirmen. Auf diese Weise wird verhindert, daß ein großer Teil der Strahlung zur nutzlosen Überdosierung des peripheren Be reichs führt. Dieser - vergleichsweise große - Teil der Strahlung wird statt dessen in den Abschirmelementen absorbiert und ist damit nutzlos.

Mit der Vorrichtung nach der DE-PS-2 358 652 lassen sich mehrere Produkte mit unterschiedlich langen Bestrahlungszeiten simultan bestrahlen. Diese Mehrzweckeigenschaft ist für die industrielle Praxis von großem Nutzen.

## Darstellung der Erfindung

Es ist Aufgabe der Erfindung, den Strahlungswirkungsgrad gegenüber dem bekannten Verfahren bzw. der bekannten Vorrichtung wesentlich zu erhöhen, ohne die vorstehend mit Mehrzweckeigenschaft bezeichnete Eigenschaften aufzugeben.

Erfindungsgemäß wird diese Aufgabe dadurch gelöst, daß das Bestrahlungsgut mindestens einmal in eine Position nahe bei der Strahlungsquelle und mindestens einmal in eine Position fern der Strahlungsquelle gebracht wird, wobei das Bestrahlungsgut in der nahen Position das Bestrahlungsgut in der fernen Position so abschirmt, daß die Abschirmwirkung in der Nähe der Symmetrieachse geringer ist als im peripheren Bereich, und das Bestrahlungsgut so bewegt wird, daß es von mindestens zwei Seiten bestrahlt wird.

Dadurch übernimmt die benachbarte Bestrahlungsguteinheit für eine bestimmte Zeit die Funktion eines Abschirmelementes, wobei die Bewegungsfrequenz so gewählt wird, daß jede Bestrahlungsguteinheit mindestens einmal die Funktion eines Abschirmelementes übernimmt. Damit wird auch die in den Abschirmeinheiten absorbierte Strahlung ausgenutzt, da ja Bestrahlungsgut vorübergehend die Abschirmeinheiten bildet.

Erfindungsgemäß wird deshalb eine Vorrichtung vorgeschlagen, die dadurch gekennzeichnet ist, daß um eine Strahlungsquelle mindestens vier das Bestrahlungsgut enthaltende Transporteinheiten auf mindestens vier Aufnahmevorrichtungen angeordnet sind und mindestens zwei Transporteinheiten in einer Position nahe zur Strahlungsquelle und mindestens zwei Transporteinheiten in einer fernen Position zur Srahlenquelle so angeordnet sind, daß das Bestrahlungsgut in der nahen Position das Bestrahlungsgut in der fernen Position so abschirmt, daß die Abschirmwirkung in der Mitte des Bestrahlungsgutes geringer ist als im peripheren Bereich.

Als Strahlungsquelle kommen beliebige für den jeweiligen Zweck brauchbare ionisierende Strahlung emittierende Quellen und insbesondere Radionuklide oder Röntgen-Strahlungsquellen in Betracht.

Weiterbildungen der Erfindung sind in den Unteransprüchen angegeben:

Besonders gute Ergebnisse werden gemäß Anspruch 2 erzielt, wenn man das Bestrahlungsgut von vier Seiten bestrahlt. Ferner kann man das Bestrahlungsgut auch kontinuierlich, schrittweise oder pendelnd drehen (Ansprüche 7 bis 9), so daß das Bestrahlungsgut auch aus mehr als vier Richtungen bestrahlt wird.

Bei kleinen Bestrahlungsanlagen kann man die Transporteinheiten einzeln nacheinander oder in Gruppen gleichzeitig bewegen. Bei größeren Bestrahlungsanlagen ist es zweckmäßig, mehrere Transporteinheiten dicht nebeneinander oder übereinander anzuordnen. Dabei kann jede Transporteinheit separat durch eine Aufnahmevorrichtung unterstützt werden. Eine derartige Anordnung wird hier als Säule bezeichnet. Eine vertikale Säule kann beispielsweise aus einem schmalen Regal mit mehreren Plätzen bestehen, auf denen Paletten mit Bestrahlungsgut übereinander abgestellt sind. Die vertikale Symmetrieachse der Säulen wird als Säulendrehachse oder Drehachse bezeichnet, auch dann, wenn die Säule nur von einer Transporteinheit gebildet wird. Sehr gute Ergebnisse werden erzielt, wenn die Säulendrehachsen paralell zu einer im ganzen als stabförmig anzusehenden Strahlungsquelle verlaufen und/oder mit der Förderrichtung übereinstimmen, mit der das Bestrahlungsgut an einer beliebigen Strahlungsquelle vorbeigefördert wird, da dieses Verfahren in der Wirkung einer stabförmigen Strahlungsquelle gleichkommt. Die vertikale Orientierung der Säulendrehachse ist dabei nicht zwingend. Die Drehachse kann beliebig im Raum orientiert sein.

Der Strahlungswirkungsgrad und die Dosishomogenität werden durch die Art und Weise, wie das Bestrahlungsgut in die beiden Positionen zur Strahlungsquelle gebracht werden, nicht nennenswert beeinflußt. Es gibt zwei grundsätzliche Transportmethoden. Eine besonders einfache Methode besteht darin, die Bestrahlungsgut auf feste, unbewegliche Aufnahmevorrichtungen, beispielsweise Regalplätze, zu stellen. Das Bestrahlungsgut kann dann beispielsweise mit einem automatischen Regalbedienungsgerät in erfindungsgemäßer Weise umgeschichtet werden. Das Bestrahlungsgut muß also auf die nahe und ferne Position gesetzt, gedreht und je nach der geometrischen Gestalt der Strahlungsquellen auch in Richtung der Säulendrehachse gefördert werden. Wegen der großen Zahl der Bewegungsabläufe ist diese Verfahren für große Durchsätze nicht besonders geeignet. Eine andere Methode besteht darin, Säulen mit mehreren Transporteinheiten in die beiden Endpositionen zu bewegen. Dabei kann mindestens die Hälfte der Säulen gleichzeitig bewegt werden. In diesen Positionen oder in leicht erreichbaren Hilfspositionen können die Säulen auch um ihre Symmetrieachse gedreht werden.

Ein einfaches Verfahren, die beiden Endpositionen zu erreichen, besteht darin, daß man die Säulen in radialer Richtung (Anspruch 3) zur Strahlungsquelle, beispielsweise auf Schienen, hin und her bewegt. Dieses Verfahren läßt sich besonders einfach modifizieren. Beispielsweise lassen sich die Endpositionen und die Bewegungsabläufe leicht verändern und individuell steuern.

Gemäß einer weiteren im Anspruch 4 gekennzeichneten Ausgestaltung der Erfindung lassen sich die Säulen auch in einer solchen Bewegung zu den beiden Endpositionen bringen, die eine Bewegungskomponente in Umfangsrichtung aufweist. Bei diesem Verfahren können alle Säulen gleichzeitig bewegt werden. Es ist daher ein besonders hoher Durchsatz möglich. Die Bewegung läßt sich so steuern, daß das Bestrahlungsgut von einer ortsfesten Fördereinrichtung be- und entladen wird. Die Bewegung kann hin- und hergehend sein oder auf einer Endlosschleife in einer Richtung ausgeführt werden. Die Endlosschleife kann auch einen Umweg enthalten, der es ermöglicht, das Bestrahlungsgut außerhalb der Abschirmung zu be- und entladen. Eine geeignete technische Ausführungsform ist beispielsweise eine an sich bekannte Einschienenhängebahn.

Gemäß einer weiteren Ausgestaltung der Erfindung lassen sich die Einrichtungen zum radialen Verschieben und zum Drehen auch auf einem Karusell anordnen. Mit diesem Karusell kann man zum Be- und Entladen eine ortsfeste Fördereinrichtung ansteuern. Mit einem speziellen Karusell läßt sich auch erreichen, daß das Bestrahlungsgut auf der fernen Position relativ zum Bestrahlungsgut auf der nahen Position in Umfangsrichtung bewegt wird. Eine solche Ausführungsform besteht beispielsweise darin, die Säulen auf der fernen Position auf einer drehbaren Plattform schrittweise auf die benachbarte Position zu bewegen. Dadurch erreicht man auch bei sehr unterschiedlich schweren Bestrahlungsgütern einen weitgehend konstanten Mittelwert der Abschirmwirkung durch die nahe zur Strahlungsquelle stehenden Säulen.

Die Anzahl der Säulen, die um eine Strahlungsquelle angeordnet werden, ist an sich beliebig. Aus praktischen Gründen ist es jedoch eine gerade Anzahl, vorzugsweise 4 bis 20 Einheiten bevorzugt. Auch die Querschnittsform der Transporteinheiten kann beliebig gewählt werden. Besonders geeignet sind die üblichen rechteckigen Transporteinheiten, insbesondere die Einheiten mit quadratischem Querschnitt.

Für einen kompletten Bestrahlungsvorgang muß das Bestrahlungsgut mindestens eine Position nahe der Strahlungsquelle und mindestens eine Position fern von der Strahlungsquelle einnehmen und mindestens von zwei Seiten bestrahlt werden. Für einen kompletten Bestrahlungsvorgang können auch mehrere derartige Bewegungszyklen ausgeführt werden. Die Zyklusfrequenz wählt man zweckmäßigerweise groß, wenn Produkte mit sehr unterschiedlicher aufzunehmender Dosis und/oder Dichte simultan bestrahlt werden sollen. Die Zyklusdauer muß aber nicht konstant sein. Besonders vorteilhaft ist es, wenn man die Zykluszeit so steuert, daß zwischen zwei Entnahmen von Bestrahlungsgut aus einer beliebigen Säule gerade ein Bewegungszyklus ausgeführt wird. Bei einer langen Strahlungsquelle mit geeigneter Aktivitätsverteilung kann jeweils das gesamte Bestrahlungsgut einer Säule be- und entladen werden. Einen höheren Wirkungsgrad erreicht man jedoch mit einer kürzeren Strahlungsquelle, wenn dafür gesorgt wird, daß das Bestrahlungsgut die Säule in Richtung der Drehachse kontinuierlich oder schrittweise mit einem Regalbediengerät vorgenommen werden. Die Säulendrehachse ist vorzugsweise vertikal im Raum orientiert. Sie kann aber auch horizontal angeordnet sein. Das erleichtert die kontinuierliche Förderung des Bestrahlungsgutes beispielsweise mit Hilfe von Rollenbahnen.

Einen besonders hohen Wirkungsgrad erhält man, wenn das Bestrahlungsgut so angeordnet wird, daß in der Nähe der Säulendrehachse ein Hohlraum (Anspruch 14) entsteht. Einen ähnlich hohen Wirkungsgrad und eine ausgezeichnete Dosishomogenität erhält man dadurch, daß man das Bestrahlungsgut so anordnet, daß im äußeren Bereich des Querschnittes eine hohe Dichte und in der Nähe der Säulendrehachse eine geringere Dichte des Bestrahlungsgutes vorhanden ist (Anspruch 15). Eine geringere Dichte in der Mitte kann man beispielsweise durch eine Verpackung des gleichen Produktes, jedoch mit größeren Lücken erreichen. Diese Maßnahmen haben einen besonders starken Effekt bei großvolumigen Transporteinheiten mit hoher Dichte.

Die auch ohne besondere Maßnahmen schon recht gute Dosishomogenität kann man bei viereckigen Säulenquerschnitten durch besondere Verfahrensmaßnahmen und Modifizierung der Vorrichtung noch weiter verbessern. Die Anwendung dieser Verbesserung ist besonders vorteilhaft, wenn Bestrahlungsgut mit sehr verschiedenen Dichten simultan bestrahlt werden soll. Als besondere Verfahrensmaßnahmen können folgende Schritte zur Verbesserung der Dosishomogenität führen:

— eine pendelnde Drehbewegung der Säule um ein Dosisminimum an den Ecken zu vermeiden.

— die Wahl einer ungleichförmigen Drehgeschwindigkeit, um die Ecken länger in größere Nähe zur Strahlungsquelle zu bringen

— die Veränderung der Endposition um die richtige Abschirmwirkung einzustellen

— die Einrichtung von mehreren Zwischenstopps oder einer veränderbaren Verschiebegeschwindigkeit auf der Bahn, um die Abschirmwirkung zu dosieren

Durch entsprechendes Modifizieren der Vorrichtung kann die Dosishomogenität weiter gesteigert werden, insbesondere durch zusätzliche Abschirmeinrichtungen oder durch eine besondere Verteilung der Aktivität über den Querschnitt der Strahlungsquelle. Eine besonders vorteilhafte Aktivitätsverteilung erhält man dadurch, daß eine symmetrische n-eckige Sruktur verwendet wird, wobei "n" der Anzahl der Säulen, einem ganzzahligen Teil davon oder einem ganzzahligen Vielfachen entspricht (Anspruch 24). Günstig ist auch eine kontinuierliche oder schrittweise Drehbewegung der Strahlungsquelle. Die Dosishomogenität und Wirkungsgrad lassen sich auch dadurch verbessern, daß man die Einzelelemente der Strahlungsquelle in Teilgruppen anordnet und diese zueinander bewegt. Dies ist insbesondere dann vorteilhaft, wenn das Bestrahlungsgut sehr nahe an der Strahlungsquelle plaziert ist.

Ferner können bevorzugt gemäß Anspruch 26 zusätzliche Abschirmeinrichtungen so angeordnet sein, daß sie die Strahlung seitlich der Linie von Strahlungsquelle und Säulendrehachse zusätzlich schwächen. Die als Blech ausführbaren Abschirmeinrichtungen können mit der Säule drehbar angeordnet oder fest zwischen Strahlungsquelle und Säule angebracht sein. Sie können der nahen Position der Säulen und/oder der fernen Position zugeordnet sein. In Zusammenwirkung mit der pendelnden Drehbewegung der Säulen sind auch bewegliche Abschirmeinrichtungen vorteilhaft. Bei der Bestrahlung eines täglich wechselnden unterschiedlichen Produktsortiments ist es besonders vorteilhaft, als Abschirmeinrichtung eine Vielzahl dünnwandiger eng beieinanderstehender Rohre zu verwenden, die je nach den gerade zu bestrahlenden Produkten, mit einer Flüssigkeit gefüllt werden. Auf diese Weise läßt sich Ort und Gestalt der wirksamen zusätzlichen Abschirmung rasch ändern.

Gemäß einer weiteren Ausgestaltung der Erfindung kann gemäß Anspruch 11 zur Einstellung der optimalen Bestrahlungsparameter eine Programmsteuerung vorgesehen werden, die alle Bewegungsabläufe veranlaßt. Besonders vorteilhaft ist eine flexible Steuerung durch eine Prozeßrechenanlage, die einen Algorithmus zur Vorausberechnung der Dosis enthält, wobei die benachbarten Säulen und die Abschirmeinrichtungen mit einbezogen werden. Je nach vorgegebener Priorität kann dann die Anlage so gesteuert werden, daß die beste Dosishomogenität, der beste Wirkungsgrad oder ein Optimum aus beiden erreicht wird. Die Steuerung kann auch durch Messung der Dosisleistung an geeigneten Orten unterstützt werden.

## Kurze Beschreibung der Zeichung

Die Erfindung wird nachstehend anhand von Ausführungsbeispielen unter Bezugnahme auf die Zeichnung näher beschrieben, in der zeigen:

Fig. 1 und 2 eine Vorrichtung mit radialer Verschieberichtung und 8 Säulen zu unterschiedlichen Zeitpunkten,

Fig. 3 den in Fig. 2 dargestellten Schnittverlauf als Karusell-Anordnung,

Fig. 4 und 5 eine Vorrichtung mit 4 Säulen zu unterschiedlichen Zeitpunkten,

Fig. 6 eine Vorrichtung mit Einschienenbahn,

Fig. 7 eine Anordnung von 12 Säulen und einer drehbaren Plattform für 6 Säulen.

Fig. 8 eine Anordnung mit einem Hohlraum in der Mitte des Bestrahlungsgutes,

Fig. 9 eine Transporteinheit aus Fig. 8.

Fig. 10 eine Anordnung mit verschiedenen Abschirmeinrichtungen,

Fig. 11 und 12 Details der Abschirmeinrichtung aus Fig. 10

Fig. 13 und 14 einen Ausschnitt aus einer Vorrichtung mit 8 Säulen und beweglichen Abschirmeinrichtungen zu unterschiedlichen Zeitpunkten.

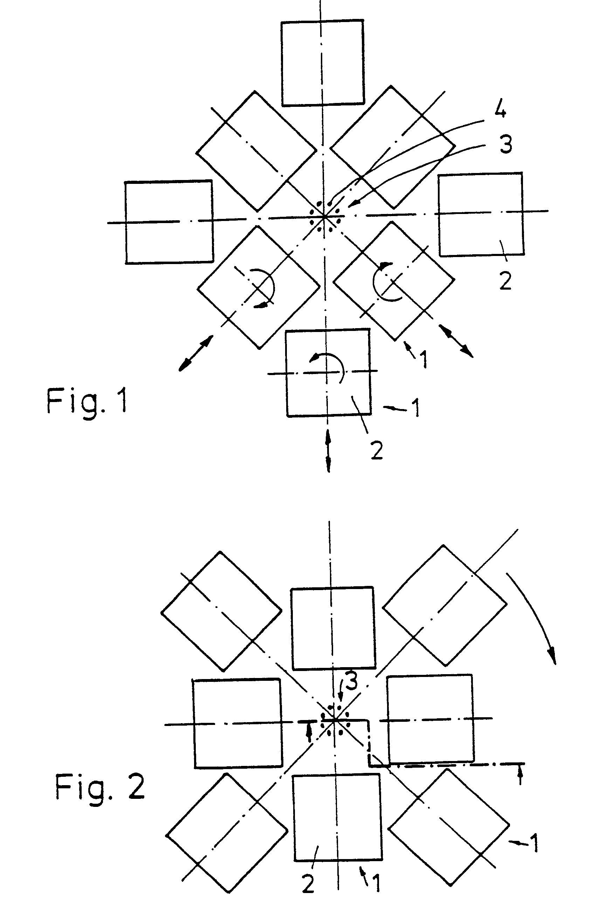

Fig. 1 zeigt eine stark vereinfachte Aufsicht auf acht vertikal angeordnete Säulen 1 mit Bestrahlungsgut 2 und einer stabförmigen Strahlungsquelle 3, die aus mehreren Einzelelementen 4, beispielsweise aus Stäben mit Co-60, zusammengesetzt ist. Die Hälfte der Säulen ist relativ nahe an die Strahlungsquelle herangeschoben und schirmt dadurch mindestens teilweise die weiter außen befindlichen Säulen ab. Durch schrittweise Drehung um 90 Grad werden alle vier Seiten jeder Säule zur Strahlungsquelle gedreht. Für weniger hohe Anforderungen an die Dosishomogenität genügt eine schrittweise Drehung um 180 Grad. Um für die Drehung ausreichend Platz zu haben, kann ein Teil oder alle Säulen kurzzeitig ein wenig nach außen in eine Zwischenposition geschoben werden. Danach werden die Plätze gewechselt, d.h., die innenliegenden Säulen werden nach außen geschoben und die außenliegenden Säulen nach innen. Die Bewegungsrichtung ist durch Pfeile angedeutet.

Fig. 2 zeigt die in Fig. 1 dargestellte Vorrichtung nach der Bewegung der Säulen. Auch in dieser Position werden die Säulen wie oben beschrieben gedreht. Bei der Drehung ist ein gegenläufiger Drehsinn zweckmäßig. Nach obiger Beschreibung hat jede Säule für einen kompletten Bestrahlungsvorgang zwei Umdrehungen ausgeführt und ist einmal in radialer Richtung hin- und herbewegt worden. Man kann aber auch so verfahren, daß man die Säulen nach jeder 90 Grad Drehung hin- und herbewegt. Dann hat jede Säule am Ende der Bestrahlung nur eine Umdrehung ausgeführt.

Zur einfacheren Be- und Entladung von Bestrahlungsgut kann man die ganze technische Einrichtung zum Halten und Bewegen des Bestrahlungsgutes auf einem Karusell anordnen und dieses dann jeweils in die gewünschte Be- und Entladeposition bewegen. Zum Ausgleich ungleichmäßiger Verteilungen der Einzelelemente 4 der Strahlungsquelle kann das Karusell auch kontinuierlich oder pendelnd gedreht werden.

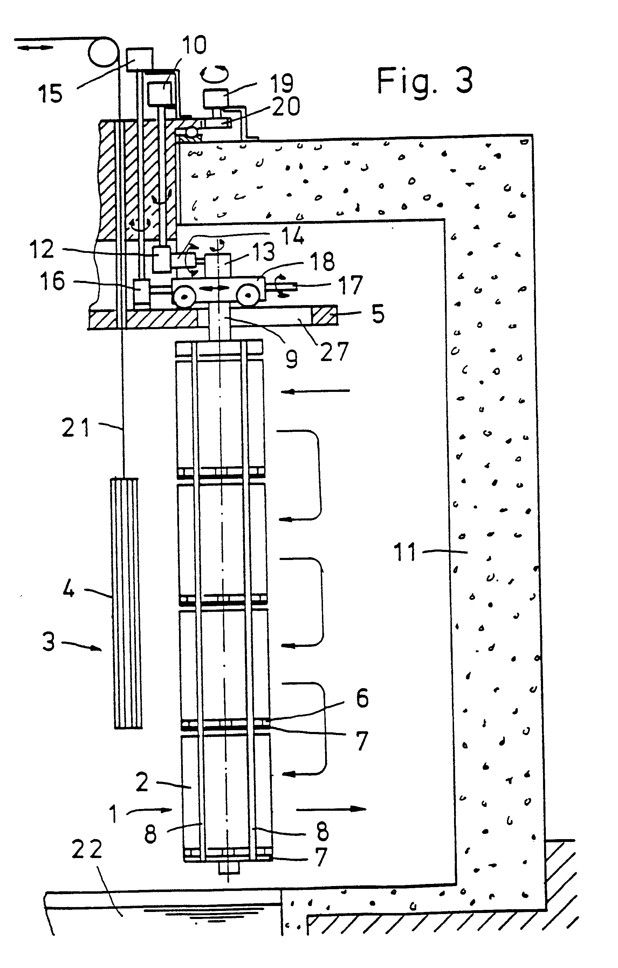

Fig. 3 zeigt einen Längsschnitt durch die in Fig. 2 dargestellte Vorrichtung. Dargestellt ist ein Karusell mit obenliegender Plattform 5 und einer Säule 1 mit Bestrahlungsgut 2, das auf Paletten 6 ruht und auf 4 separaten Aufnahmevorrichtungen 7 abgestellt ist. Die Aufnahmevorrichtungen 7 werden durch außenliegende Träger 8 gehalten, die an einer drehbar gelagerten Achse 9 befestigt sind. Die mögliche Linearbewegung der Achse 9 wird durch den Schlitz 27 begrenzt. Der Antrieb 10 für diese Achse 9 liegt außerhalb der Abschirmung 11. Die Drehbewegung von Antrieb 10 wird über zwei Kegelradgetriebe 12 und 13 auf die Säule 1 übertragen. Die Übertragungsspindel 14 läßt sich teleskopartig ausziehen. Für die Linearbewegung der Säulen sorgt ein anderer Antrieb 15, der über ein weiteres Winkelgetriebe 16 eine Gewindespindel 17 bewegt. Diese Drehbewegung wird von einer hier nicht dargestellten Mutter in eine Linearbewegung umgewandelt, die auf den schienengeführten Wagen 18 einwirkt. Ein weiterer Antrieb 19 treibt über ein Zahnrad 20 die gesamte Plattform 5 an. Bei großen Anlagen wird meistens eine zusätzliche Lagerung am unteren Ende der Säule 1 erforderlich. Die Strahlungsquelle 3, bestehend aus einer Vielzahl von Co-60 Stäben 4, hängt an einem Stahlseil 21 und kann über ein hier nicht vollständig dargestelltes Quellenhubwerk in ein Wasserbecken 22 abgesenkt werden. Die Pfeile an der Säule 1 sollen zeigen, wie das Bestrahlungsgut 2 die Säule während des Bestrahlungsprozesses durchwandern kann. Zunächst wird also die untere Transporteinheit entnommen. Dann werden die drei verbleibenden Einheiten ein Stockwerk tiefer gesetzt. Zum Abschluß wird eine unbestrahlte Transporteinheit in das oberste Stockwerk eingestellt. Wenn sich Strahlungsquelle 3 im Wasserbecken 22 befindet, kann der Umschichtungsvorgang auch manuell durchgeführt werden.

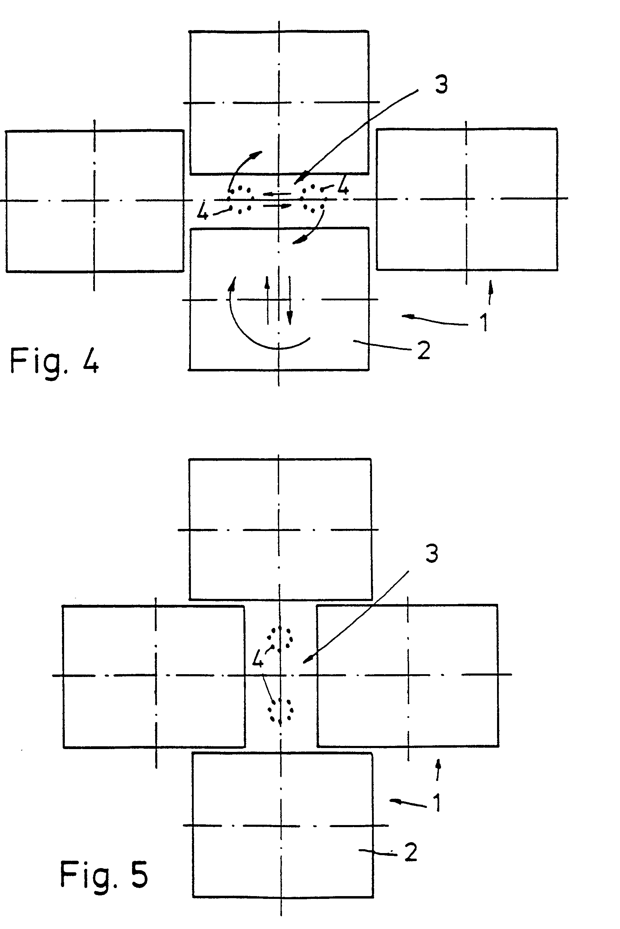

In Fig. 4 und Fig. 5 ist eine Anordnung mit vier Säulen 1 dargestellt. Die Einzelelemente 4 der Strahlungsquelle 3 sind in zwei Gruppen angeordnet, die, wie mit Pfeilen angedeutet, zueinander bewegbar sind. Außerdem ist die Strahlungsquelle 3 im ganzen drehbar. Diese Maßnahme kann zur besseren Dosishomogenität beitragen. Es ermöglicht auch eine größere Anzahl von Einzelelementen 4 im relativ kleinen Innenraum anzuordnen. Die Pfeile auf dem Querschnitt der Säule 1 sollen die hin- und hergehende Bewegung der Säule 1 und Drehbewegung darstellen. Die Fig. 5 unterscheidet sich von Fig. 4 nur durch einen anderen Bewegungszustand: Die inneren Säulen sind in eine ferne Position zur Strahlungsquelle und die äußeren in eine nahe Position zur Strahlungsquelle bewegt worden. Außerdem ist die Strahlungsquelle um 90 Grad gedreht worden. Als nächster Schritt wäre eine Drehung beispielsweise um 90 Grad zu vollziehen. Dazu müssen die Säulen 1 jedoch für kurze Zeit auf eine Zwischenposition gebracht werden, damit sie sich nicht gegenseitig behindern. Eine andere Möglichkeit, die Säulen von allen vier Seiten zu bestrahlen, besteht darin, die Säulen 1 um die Strahlungsquelle 3 in Umfangsrichtung herumzuführen, beispielsweise durch ein an sich bekanntes Ein- oder Mehrschienensystem.

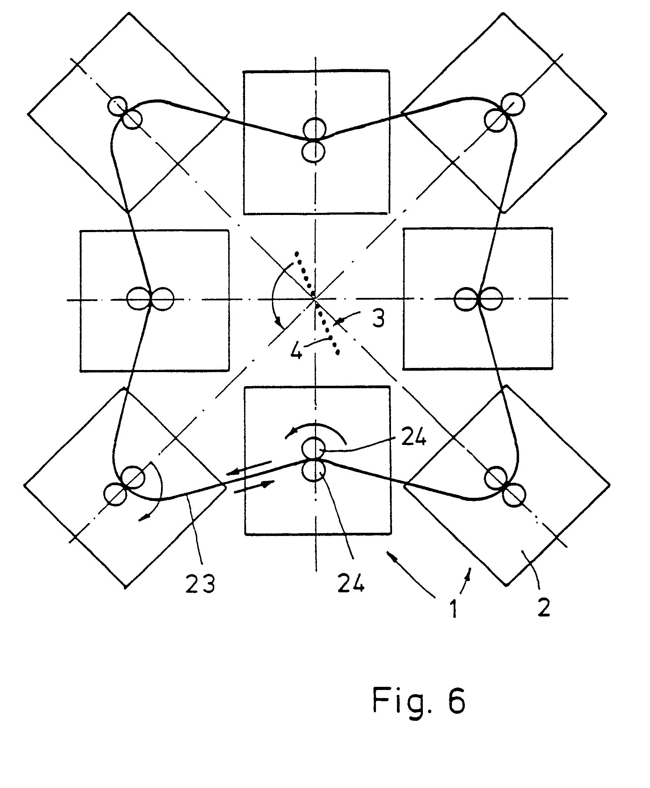

Fig. 6 zeigt den Prinzipaufbau eines Einschienensystem für eine Vorrichtung mit acht Säulen. Der Verlauf der Schiene ist durch die Linie 23 vereinfacht dargestellt. Die Säulen werden durch Führungsrollen 24 an dieser Schiene geführt. Die Säulen sind untereinander in an sich bekannter Weise durch Gelenkglieder verbunden und haben Antriebe für die Linear- und Drehbewegung. Ein besonderer Vorzug dieses Einschienensytms besteht darin, daß jede Säule durch eine entsprechende Steuerung an eine fest installierte Fördereinrichtung zum Be- und Entladen herangeführt werden kann. Die Bewegungsrichtung ist beliebig und kann während der Bestrahlung wechseln. Es kommt nur darauf an, daß für einen Bestrahlungsvorgang mindestens einmal eine Position nahe zur Strahlungsquelle und fern zur Strahlungsquelle eingenommen wird. Es können natürlich auch andere Fördereinrichtungen als Einschienenbahn benutzt werden, um diese Position zu erreichen. Mit der Form der Strahlungsquelle 3 soll deutlich gemacht werden, daß nahezu jede Form der Strahlungsquelle für das erfindungsgemäße Verfahren und die Vorrichtung geeignet ist, die in den Innenraum einer Vorrichtung hineinpaßt. Die Anordnung der Einzelelemente 4 der Strahlungsquelle entspricht einer Flächenquelle. Um Inhomogenitäten zu vermeiden, kann diese Strahlungsquelle um ihre Mittelachse gedreht werden. Eine Drehung in Schritten mit Zwischenstopps, die zwischen den Linien liegen, die die Drehachsen von Strahlungsquelle und Säulen bilden, ist hier besonders vorteilhaft, weil hier die gegenseitige Strahlenabsorption der Einzelelemente 4 sich kaum auf den Wirkungsgrad der Vorrichtung auswirkt.

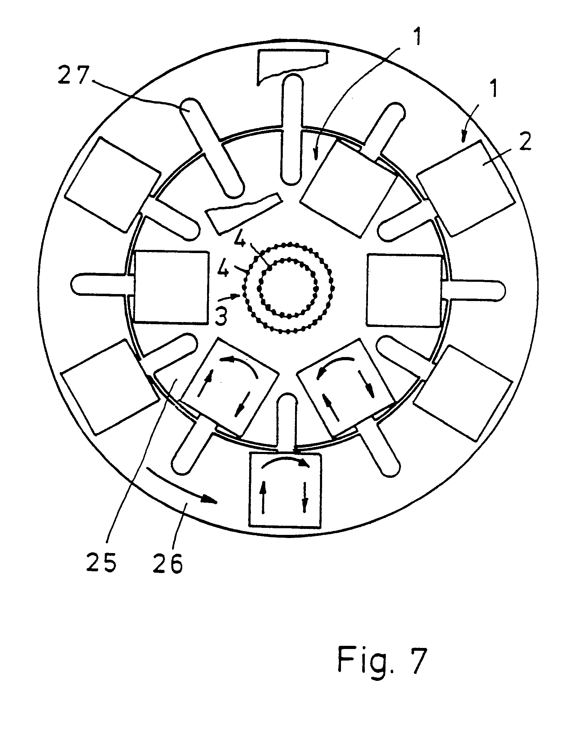

In Fig. 7 ist eine spezielle Art eines Karusells dargestellt. Diese Figur hat eine gewisse Ähnlichkeit mit Fig. 3. Die Karusellplattform ist hier aufgeteilt in einen feststehenden inneren Ring 25 und einen drehbaren äußeren Ring 26. Die Säulen 1 werden nun zwischen diesen beiden Ringen in radialer Richtung hin- und hergeschoben. Die Schlitze 27 geben die maximal erreichbaren inneren und äußeren Positionen an. Die äußere Position ist so gewählt, daß sich die Säulen 1 bei der kurzen Drehbewegung der Plattform 5 nicht behindern. Bei der Bestrahlung können diese Säulen ganz dicht an die nahe zur Strahlungsquelle stehenden Säulen herangeschoben werden. Die Strahlungsquelle 3 ist hier als doppelreihige Käfiganordnung dargestellt.

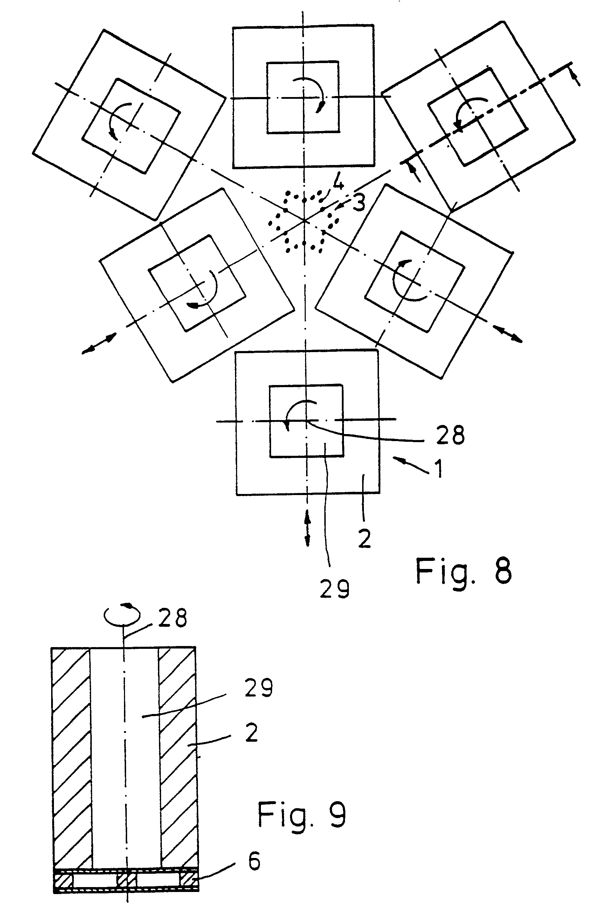

In Fig. 8 ist eine Anordnung mit sechs Säulen 1 dargestellt, die um eine Strahlungsquelle 3 mit sternförmigem Querschnitt angeordnet sind. Die Säulen haben im Bereich ihrer Drehachse 28 einen Hohlraum 29.

Fig. 9 zeigt diesen Hohlraum im Längsschnitt durch eine Transporteinheit. Das Bestrahlungsgut 2 ist in Form einer Wand angeordnet und steht auf einer Palette 6. Die Säulen 1 enthalten vorzugsweise mehrere solcher Transporteinheiten. Vorzugsweise sind alle Transporteinheiten einer Säule mit einem Hohlraum versehen. Das Bestrahlungsgut der benachbarten Säulen 1 kann ohne Hohlraum angeordnet sein und auch eine größere oder kleiner Dichte haben.

Anstelle des in Fig. 8 und 9 gezeigten Hohlraums 29 kann man auch Bestrahlungsgut mit einer geringen Dichte im Bereich um die Drehachse 28 herum und im peripheren Bereich Bestrahlungsgut mit einer hohen Dichte anordnen. Der radial zur Drehachse 28 verlaufende Dichtegradient kann kontinuierlich oder in Stufen mit zunehmendem Abstand von der Drehachse veränderbar sein. Diese Maßnahme hat einen günstigen Einfluß auf den Wirkungsgrad und die Dosishomogenität.

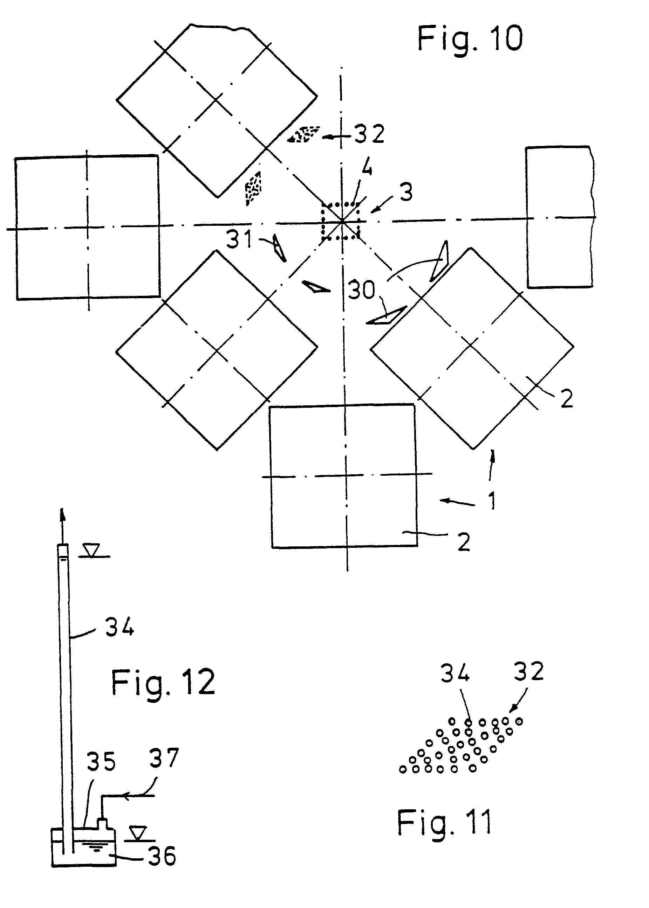

In Fig. 10 ist ein Ausschnitt aus einer Anordnung mit acht Säulen 1 dargestellt, die verschiedene Varianten von zusätzlichen Abschirmeinrichtungen 30, 31, 32 aufweist. Die Abschirmeinrichtungen 30, 31, 32 verlaufen parallel zur Säulendrehachse und sind in etwa so lang wie die Säulen. Mit diesen Abschirmeinrichtungen kann die, auch ohne diese zusätzlichen Einrichtungen schon sehr gute Dosishomogenität, noch weiter verbessert werden. Man ordnet diese Abschirmeinrichtungen 30, 31, 32 zweckmäßig so an, daß sie nur dort den Strahlengang zusätzlich schwächen, wo erfahrungsgemäß eine Überdosierung auftreten würde. Die Form dieser Einrichtungen muß an die Größe der Transporteinheit, deren Dichte, der Strahlungsquellengeometrie und den Plazierungsort angepaßt werden. Die Abschirmeinrichtungen 30 sind in der nahen Position der Säulen sehr dicht am Bestrahlungsgut 2 angeordnet. Um eine Drehbewegung der Säulen 1 durchzuführen, muß diese kurzzeitig radial nach außen geschoben werden, oder die Abschirmeinrichtungen 30 müssen nach innen geschoben werden. Die Abschirmeinrichtungen 31 sind dagegen so weit im Innenraum angeordnet, daß eine ungehinderte Drehbewegung der Säulen an ihren Endpositionen möglich ist. Die Abschirmeinrichtungen 32 bestehen aus einer Vielzahl dünnwandiger Rohre 34. Diese Form und Wirkungsweise ist in Fig. 11 und 12 ausführlich dargestellt. Einen Teil dieser Rohre 34 kann man beispielsweise mit Wasser oder Quecksilber füllen und so die Abschirmwirkung sehr schnell auf das zu bestrahlende Produkt abstimmen. In begrenztem Umfang läßt sich so eine Formveränderlichkeit und eine Ortsveränderlichkeit simulieren. Die Versorgung des Rohres 34 kann aus einem Behälter 35 geschehen, der eine Flüssigkeit 36 enthält, in der das Rohr 34 eintaucht. Mit Hilfe von Luft aus Leitung 37 läßt sich dann das Rohr 34 in einfacher Weise füllen und durch Entspannen wieder entleeren.

Man kann die Abschirmeinrichtungen 30, 31, 32 auch vor den Säulen anordnen, die in der Position fern zur Strahlungsquelle stehen. In diesem Fall sind dann die Abschirmeinrichtungen beweglich auszuführen. Die Beweglichkeit ist nicht erforderlich, wenn eine Anordnung nach Fig. 6 gewählt wird.

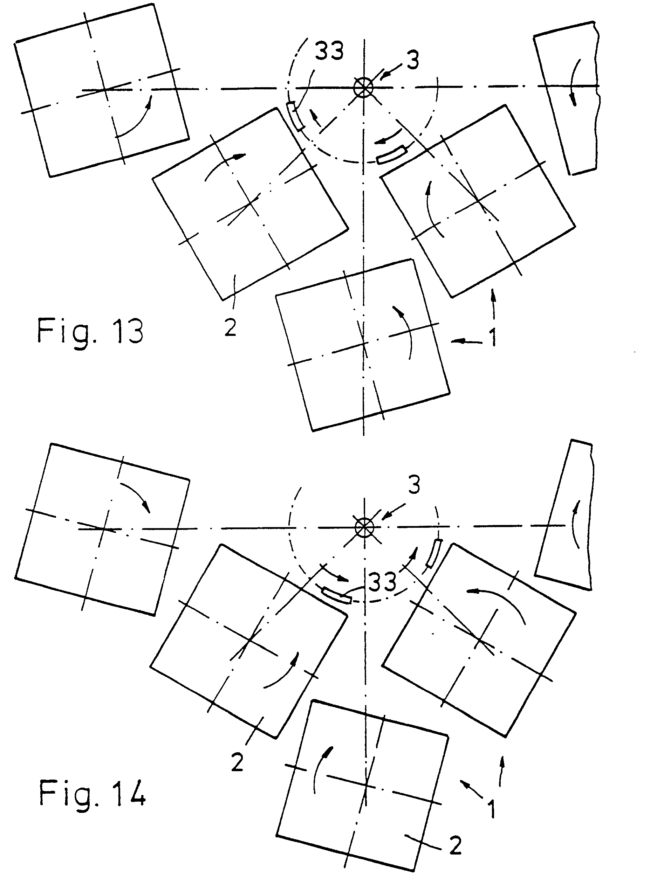

Fig. 13 und 14 zeigen einen Ausschnitt aus einer Vorrichtung mit acht 8 Säulen 1 in einem unterschiedlichen Bewegungszustand bei einer pendelnden Drehbewegung der Säulen 1.

Fig. 13 zeigt den Zustand nach einer Rechtsdrehung der innenliegenden Säulen in einem kleinen Winkel. In diesem Zustand befindet sich in der Nähe der am weitesten nach innen ragenden Ecke eine Abschirmeinrichtung 33, deren Wirkung nicht nur auf die Ecke beschränkt ist, sondern auch den peripheren Bereich der dahinterliegenden Seitenpartie beeinflußt. Diese Schwächung wirkt sich auch auf die außenliegenden Säulen aus. Um alle Seitenpartien in gleicher Weise zu bestrahlen, werden nun die innenliegenden Säulen ein wenig nach links gedreht. Gleichzeitig werden auch die Abschirmeinrichtungen 33 auf einer Kreisbahn um die Strahlungsquellenachse so gedreht, daß sie nun wieder vor der am weitesten nach innen liegenden Ecke der Säule 1 angeordnet sind.

Fig. 14 zeigt diesen Zustand. Dieser Vorgang wiederholt sich für jede 90 Grad oder 180 Grad Drehung. Auf diese Weise erreicht man auch für sehr schwere Produkte eine ausgezeichnete Dosishomogenität. Abschirmeinrichtungen sind auch dann vorteilhaft, wenn das Bestrahlungsgut, wie bei Fig. 8 und 9 beschrieben, auf besondere Weise gepackt ist.

Die vorliegende Erfindung ist besonders für größere industrielle Bestrahlungsanlagen geeignet. Die Größe der Transporteinheiten liegt vorzugsweise zwischen 0,1m und 20m. Große Einheiten eignen sich besonders gut zur Bestrahlung von Produkten mit geringer Dichte. So wurde beispielsweise für eine 8m große würfelförmige Einheit mit einer Dichte von 0,15g/cm ein Wirkungsgrad von über 50 % ermittelt. Dabei kann ein Verhältnis von maximaler Dosis zu minimaler Dosis von ca. 1, 15 bis 1,3 erreicht werden. Bei höherer Dichte und geeigneter Art und Größe der Transporteinheiten kann der Wirkungsgrad auch über 60 % liegen. Ein besonderer Vorteil der Erfindung besteht darin, daß Produkte mit unterschiedlicher Dosis und/oder Dichte simultan bestrahlt werden können.

**Patentansprüche**

1. Verfahren zur Bestrahlung von Bestrahlungsgut mit einer ionisierender Strahlung insbesondere einer Röntgenoder Gamma-Strahlungsquelle, wobei des Bestrahlungsgut zu Transporteinheiten verpackt ist, die eine Symmetrieachse besitzen, die paraleel zu einer Förderrichtung oder paraleel zur längsten Ausdehnung der Strahlungsquelle verläuft, dadurch *gekennzeichnet*, daß das Bestrahlungsgut mindestens einmal in eine Position nahe bei der Strahlungsquelle und mindestens einmal in eine Position fern der Strahlungsquelle gebracht wird, daß das Bestrahlungsgut in der nahen Position das Bestrahlungsgut in der fernen Position derart abschirmt, daß die Abschirmwirkung in der Nähe der Symmetrieachse geringer ist als im peripheren Bereich und, daß das Bestrahlungsgut so bewegt wird, daß es von mindestens zwei Seiten bestrahlt wird.

2. Verfahren nach Anspruch 1, dadurch *gekennzeichnet*, daß das Bestrahlungsgut von mindestens vier Seiten bestrahlt wird.

3. Verfahren nach Anspruch 1, dadurch *gekennzeichnet*, daß das Bestrahlungsgut in radialer Richtung zur Strahlungsquelle zu den einzelnen Positionen bewegt wird.

4. Verfahren nach Anspruch 1, dadurch *gekennzeichnet*, daß das Bestrahlungsgut in Umfangsrichtung zu den einzelnen Positionen bewegt wird.

5. Verfahren nach Anspruch 1,dadurch *gekennzeichnet*, daß das Bestrahlungsgut die Vorrichtung in mindestens zwei feststehenden Säulenpositionen durchwandert.

6. Verfahren nach Anspruch 1, dadurch *gekennzeichnet*, daß das Bestrahlungsgut auf der fernen Position relativ zum Bestrahlungsgut auf der nahen Position in Umfangsrichtung bewegt wird.

7. Verfahren nach Anspruch 1, dadurch *gekennzeichnet*, daß das Bestrahlungsgut kontinuierlich gedreht wird.

8. Verfahren nach Anspruch 1, dadurch *gekennzeichnet*, daß das Bestrahlungsgut schrittweise gedreht wird.

9. Verfahren nach Anspruch 1, dadurch *gekennzeichnet*, daß das Bestrahlungsgut pendelnd gedreht wird.

10. Verfahren nach Anspruch 1, dadurch *gekennzeichnet*, daß die Position des Bestrahlungsgutes während der Bestrahlung kontinuierlich oder schrittweise verändert wird.

11. Verfahren nach Anspruch 1, dadurch *gekennzeichnet*, daß die Bewegungsabläufe von einer Prozeßrechenanlage gesteuert werden, die zur Steuerung der Bewegunsabläufe die an den einzelnen Positionen absorbierte Dosis berechnet.

12. Verfahren nach Anspruch 1, dadurch *gekennzeichnet*, daß die Zykluszeit jeweils mit der Zeit bis zur nächsten Entnahme von Bestrahlungsgut in Übereinstimmung gebrach wird.

13. Verfahren nach Anspruch 1, dadurch *gekennzeichnet*, daß das Bestrahlungsgut in Richtung der Säulenachse schrittweise oder kontinuierlich gefördert wird.

14. Verfahren nach Anspruch 1, dadurch *gekennzeichnet*, daß das Bestrahlungsgut so angeordnet wird, daß in der Nähe der Säulendrehachse ein Hohlraum entsteht.

15. Verfahren nach Anspruch 1, dadurch *gekennzeichnet*, daß in der Nähe der Säulendrehachse eine geringere Dichte vorhanden ist als im peripheren Bereich des Säulenquerschnitts.

16. Vorrichtung zur Durchführung des Verfahrens nach einem der Ansprüche 1 bis 15, dadurch *gekennzeichnet*, daß um eine Strahlungsquelle mindestens vier das Bestrahlunsgut enthaltende Transporteinheiten auf mindestens vier Aufnahmevorrichtungen angeordnet sind, und daß mindestens zwei der Transporteinheiten in einer Position nahe bei der Strahlungsquelle und mindestens zwei der Transporteinheiten in einer zur Strahlungsquelle fernen Position derart angeordnet sind, daß das Bestrahlungsgut in der nahen Position das Bestrahlungsgut in der fernen Position so abschirmt, daß die Abschirmwirkung in der Mitte des Bestrahlungsgutes geringer ist als im peripheren Bereich.

17. Vorrichtung nach Anspruch 16, dadurch *gekennzeichnet*, daß die Aufnahmevorrichtungen drehbar angeordnet sind.

18. Vorrichtung nach Anspruch 16, dadurch *gekennzeichnet*, daß die Aufnahmevorrichtungen radial zur Strahlungsquelle verschiebbar angeordnet sind.

19. Vorrichtung nach Anspruch 16, dadurch *gekennzeichnet*, daß die Aufnahmevorrichtungen in Umfangsrichtung verschiebbar angeordnet sind.

20. Vorrichtung nach Anspruch 16, dadurch *gekennzeichnet*, daß die Aufnahmevorrichtungen so angeordnet sind, daß sie auf einer Linie bewegbar sind, die sowohl eine radiale Richtungskomponente als auch eine Richtungskomponente in Umfangsrichtung aufweist.

21. Vorrichtung nach Anspruch 16, dadurch *gekennzeichnet*, daß mindestens ein Teil der Aufnahmevorrichtung auf einem Karusell angeordnet ist.

22. Vorrichtung nach Anspruch 16, dadurch *gekennzeichnet*, daß die Strahlungsquelle drehbar angeordnet ist.

23. Vorrichtung nach Anspruch 16, dadurch *gekennzeichnet*, daß die Einzelelemente der Strahlungsquelle in Teilgruppen angeordnet sind, die zueinander verschiebbar sind.

24. Vorrichtung nach Anspruch 16, dadurch *gekennzeichnet*, daß der Querschnitt der Strahlungsquelle eine symmetrische n-förmige Struktur besitzt, wobei "n" der Anzahl der nebeneinander angeordneten Aufnahmevorrichtungen, einem ganzzahligen Teil davon oder einem ganzzahligen Vielfachen entspricht.

25. Vorrichtung nach Anspruch 16, dadurch *gekennzeichnet*, daß mehrere Aufnahmevorrichtungen in Richtung der Säulendrehachse angeordnet sind.

26. Vorrichtung nach Anspruch 16, dadurch *gekennzeichnet*, daß zwischen der Strahlungsquelle und mindestens einer Aufnahmevorrichtung zusätzliche Abschirmeinrichtungen derart angeordnet sind, daß die Strahlung seitlich einer Verbindungslinie von der Mitte der Strahlungsquelle zur Mitte des Bestrahlungsgutes zusätzlich geschwächt wird.

27. Vorrichtung nach Anspruch 26, dadurch *gekennzeichnet*, daß die Abschirmeinrichtungen ortsveränderlich angeordnet sind oder eine gleichartige Wirkung besitzen.

28. Vorrichtung nach Anspruch 26, dadurch *gekennzeichnet*, daß die Abschirmeinrichtungen formveränderlich sind.

## Claims

1. A process for irradiating objects by means of an ionizing radiation especially an x-ray or a gamma-radiation source whereby the objects are packaged in shipping units having a symmetrical axis extending parallel to a direction of conveyance or parallel to the longest extension about a source of radiation wherein the objects to be irradiated are brought at least one time to a position near the source and at least one time to a position at a distance from the source, whereby' the objects in the near position shield the objects in the distant position in such a manner that the shielding effect near the symmetrical axis is less than in the peripheral area and wherein the objects are moved in such a manner that they are irradiated from at least two sides.

2. A process as defined in claim 1, wherein the objects are irradiated from at least four sides.

3. A process as defined in claim 1, wherein the objects are moved to their individual positions in a radial direction to the source of radiation.

4. A process as defined in claim 1, wherein the objects are moved to said individual positions in a circumferential direction.

5. A process as defined in claim 1, wherein the objects traverse the apparatus in at least two stationary columnar positions.

6. A process as defined in claim 1, wherein the objects in the distant position relative to the radiation source are moved to the near position in a circumferential direction.

7. A process as defined in claim 1, wherein the objects are continuously rotated.

8. A process as defined in claim 1, wherein the objects are rotated step by step.

9. A process as defined in claim 1, wherein the objects are rotated pendulantly.

10. A process as defined in claim 1, wherein the positions of the objects are changed continuously or step by step during irradiation.

11. A process as defined in claim 1, wherein the movement procedures are regulated by a process computer, which calculates the dosage absorbed at the individual positions in order to regulate the movement procedures.

12. A process as defined in claim 1, wherein the cycle period is made to conform each time with the time until the next removal of objects.

13. A process as defined in claim 1, wherein the objects are conveyed step by step or continuously in the direction of the axis of the column.

14. A process as defined in claim 1, wherein the objects are arranged in such a manner that an empty space is created near the axis of rotation of the column.

15. A process as defined in claim 1, wherein the density near the axis of rotation of the column is less than in the peripheral area of the cross-section of the column.

16. An apparatus for carrying out the process defined in claim 1, 2, 3, 4, 5, 6, 7, 8, 9, 10, 11, 12, 13, 14 or 15, wherein at least four shipping units containing the objects are arranged around a source of radiation on at least four carrying devices and at least two shipping units are arranged in a position near the radiation source and at least two shipping units are arranged in a position at a distance from the radiation source in such a manner that the objects in the near position shield the objects in the distant position in such a way that the shielding effect is less in the center of the objects than in the peripheral area.

17. An apparatus as defined in claim 16, wherein the carrying devices are arranged in a rotatable manner.

18. An apparatus as defined in claim 16, wherein the carrying devices are arranged in such a manner that they can be moved in a radial direction to the source of radiation.

19. An apparatus as defined in claim 16, wherein the carrying devices are arranged in such a manner that they can be moved in a circumferential direction.

20. An apparatus as defined in claim 16, wherein the carrying devices are arranged in such a manner that they can be moved on a line which has a radial direction component as well as a direction component in the circumferential direction.

21. An apparatus as defined in claim 16, wherein at least part of the carrying device is arranged on a carousel.

22. An apparatus as defined in claim 16, wherein the source of radiation is arranged in a rotatable manner.

23. An apparatus as defined in claim 16, wherein the individual elements of the radiation source are arranged in subgroups which can be moved to and fro from each other.

24. An apparatus as defined in claim 16, wherein the cross-section of the radiation source possesses a symmetrical n-shaped structure, whereby "n" is the number of carrying devices arranged side by side, an integral part thereof or an integral multiple thereof.

25. An apparatus as defined in claim 16, wherein several carrying devices are arranged in the direction of the axis of rotation of the column.

26. An apparatus as defined in claim 16, wherein additional shielding devices are arranged between the radiation source and at least one carrying device in such a manner that the radiation lateral of a flow line from the center of the radiation source to the center of the objects to be irradiated is further weakened.

27. An apparatus as defined in claim 26, wherein the shielding devices are arranged in such a manner that they can change sites or possess a similar effect.

28. An apparatus as defined in claim 26, wherein the shielding devices can change form.

**Revendications**

1. Procédé d'irradiation par un rayonnement ionisant d'un matériau à irradier, au moyen d'une source du rayonnement X ou gamma, dans lequel le matériau à irradier est emballé dans des unités de transport dont l'axe de symétrie est parallèle à un sens de transport ou parallèle à l'étendue la plus longue de la source du rayonnement, *caractérisé en ce* que le matériau à irradier est déplacé au moins une fois dans une position proche de la source du rayonnement, et au moins une fois dans une position à distance de la source du rayonnement, et en ce que le matériau à irradier, dans sa position proche, protège le matériau à irradier en position à distance, d'une façon que l'effet de blindage proche de l'axe de symétrie est plus petit que l'effet dans la zone périphérique, et en ce que le matériau à irradier est si déplacé qu'il soit irradié d'au moins deux côtés.

2. Procédé selon la Revendication 1, *caractérisé en ce* que le matériau à irradier est irradié d'au moins quatre côtés.

3. Procédé selon la Revendication 1, *caractérisé en ce* que le matériau à irradier est déplacé dans les positions individuelles en sens radial relativement à la source du rayonnement.

4. Procédé selon la Revendication 1, *caractérisé en ce* que le matériau à irradier est déplacé dans les positions individuelles en sens périphérique.

5. Procédé selon la Revendication 1, *caractérisé en ce* que le matériau à irradier est passé par le dispositif à au moins deux positions de colonnes stationnaires.

6. Procédé selon la Revendication 1, *caractérisé en ce* que le matériau à irradier est déplacé, dans sa position à distance, en sens périphérique relativement au matériau à irradier dans position proche.

7. Procédé selon la Revendication 1, *caractérisé en ce* que le matériau à irradier est tourné en continu.

8. Procédé selon la Revendication 1, *caractérisé en ce* que le matériau à irradier est tourné pas à pas.

17

9. Procédé selon la Revendication 1, *caractérisé en ce* que le matériau à irradier est tourné en rotation oscillante.

10. Procédé selon la Revendication 1, *caractérisé en ce* que la position du matériau à irradier est variée soit en continu soit pas à pas au cours de l'irradiation.

11. Procédé selon la Revendication 1, *caractérisé en ce* que les cycles de mouvement sont réglés par un système de calculation industrielle qui établit le dosage absorbé aux positions individuelles afin de régler la suite des mouvements.

12. Procédé selon la Revendication 1, *caractérisé en ce* que le temps de cycle est accordé respectivement au temps s'écoulant jusqu'au décharge du matériau à irradier.

13. Procédé selon la Revendication 1, *caractérisé en ce* que le matériau à irradier est transporté soit pas à en continu dans la direction de l'axe de la colonne.

14. Procédé selon la Revendication 1, *caractérisé en ce* que le matériau à irradier est si disposé qu'il y a un espace creux proche de l'axe de rotation de la colonne.

15. Procédé selon la Revendication 1, *caractérisé en ce* que proche de l'axe de rotation de la colonne la densité est plus basse que dans la zone périphérique de la coupe transversale par la colonne.

16. Dispositif à réaliser le procédé selon quelconque des Revendications 1 à 15, *caractérisé en ce* qu'au moins quatre unités de transport contenantes le matériau à irradier sont disposées autour une source du rayonnement sur au moins quatre moyens de positionnement, et en ce qu'au moins deux desdites unités de transport sont disposées dans une position proche de la source du rayonnement, pendant qu'au moins deux desdites unités de transport sont disposées dans une position à distance de la source du rayonnement, d'une façon que le matériau à irradier qui se trouve dans la position proche protège le matériau à irradier dans la position à distance d'une manière que l'effet de blindage au centre du matériau à irradier soit plus petit que l'effet dans la zone périphérique.

17. Dispositif selon la Revendication 16, *caractérisé en ce* que les moyens de positionnement sont disposés pour un mouvement rotatif.

18. Dispositif selon la Revendication 16, *caractérisé en ce* que les moyens de positionnement sont disposés pour un déplacement radial relativement à la source du rayonnement.

19. Dispositif selon la Revendication 16, *caracté-*

18

*risé en ce* que les moyens de positionnement sont disposés pour un déplacement en sens périphérique.

20. Dispositif selon la Revendication 16, *caractérisé en ce* que les moyens de positionnement sont si disposés qu'il se peuvent déplacer sur une ligne qui comprend un composant directionnel radial ainsi qu'un composant directionnel périphérique.

21. Dispositif selon la Revendication 16, *caractérisé en ce* qu'au moins une partie des moyens de positionnement est disposée sur un carrousel.

22. Dispositif selon la Revendication 16, *caractérisé en ce* que la source du rayonnement est disposée pour un mouvement rotatif.

23. Dispositif selon la Revendication 16, *caractérisé en ce* que les éléments isolés de la source du rayonnement sont disposés en groupes partiels qui sont déplaçables l'un relativement à l'autre.

24. Dispositif selon la Revendication 16, *caractérisé en ce* que la coupe transversale de la source du rayonnement présente une structure symétrique à $n$ coins, "$n$" correspondant au nombre des moyens de positionnement disposés l'un de côté de l'autre, à une partie paire de ce nombre ou à un multiple pair.

25. Dispositif selon la Revendication 16, *caractérisé en ce* que plusieurs moyens de positionnement sont disposés dans la direction de l'axe de rotation de la colonne.

26. Dispositif selon la Revendication 16, *caractérisé en ce* que des moyens de blindage supplémentaires sont disposés entre la source du rayonnement et au moins un des moyens de positionnement d'une façon que le rayonnement soit atténué supplémentairement au côté d'une ligne reliant le centre de la source du rayonnement avec le centre du matériau à irradier.

27. Dispositif selon la Revendication 26, *caractérisé en ce* que les moyens de blindage sont disposés pour un déplacement ou produisent un effet similaire.

28. Dispositif selon la Revendication 26, caractérisé en ce que les moyens de blindage sont variable en forme.

Fig. 1

Fig. 2

Fig. 3

Fig. 4

3

4

4

2

1

Fig. 5

3

4

1

2

Fig. 6

Fig. 7

Fig. 8

Fig. 9

Fig. 10

32

4

3

31

30

2

1

2

34

Fig. 12

35    37

36

34    32

Fig. 11

Fig. 13

Fig. 14